# EUROPEAN PATENT APPLICATION

(11) **EP 1 834 669 A1**
(43) Date of publication of application: **19.09.2007**
(21) Application number: 06005327.9
(22) Date of filing: 15.03.2006
(51) Int. Cl.: A61P 31/12, A61K 31/175

(54) **Use of specific hydrazone compounds for the treatment of viral infections**

(71) Applicant: Heinrich-Pette-Institut, 20251 Hamburg (DE)
(72) Inventor: Hauber, Joachim, 20535 Hamburg (DE); Hauber, Ilona, 20535 Hamburg (DE); Schäfer, Birgit, 22529 Hamburg (DE); Bevec, Dorian, 82110 Germering (DE)
(74) Representative: UEXKÜLL & STOLBERG

(57) **Abstract**

The present invention relates to the use of specific hydrazone compounds for the treatment of viral infections, in particular of retroviral infections and most preferably of lentiviral or oncoretroviral infections. According to a specific embodiment of the invention, the compound is 2-[4-(aminoiminomethyl)-2,3-dihydro-1H-inden-1-ylidene]-hydrazinecarboximidamide or a salt thereof, and the retroviral infection is an HIV infection, in particular HIV-1.

## Description

The present invention relates to the use of specific hydrazone compounds for the treatment of viral infections, in particular of retroviral infections and most preferably of lentiviral or oncoretroviral infections. According to a specific embodiment of the invention, the compound is 2-[4-(aminoiminomethyl)-2,3-dihydro-1H-inden-1-ylidene]-hydrazinecarboximidamide or a salt thereof and the retroviral infection is an HIV infection, in particular HIV-1.

### Background of the invention

In the 1990s, the introduction of highly active antiretroviral therapy (HAART) into clinical practice has significantly improved morbidity and mortality of HIV-infected patients (Palella, F. J., Jr., Delaney, K. M., Moorman, A. C., Loveless, M. O., Fuhrer, J., Satten, G. A., Aschman, D. J. & Holmberg, S. D. (1998) N. Engl. J. Med. 338, 853-860). Current HAART targets the viral proteins reverse transcriptase, protease and gp41. Unfortunately, long-term HAART is often accompanied by substantial toxic side effects in patients (Dybul, M., Fauci, A. S., Bartlett, J. G., Kaplan, J. E. & Pau, A. K. (2002) Ann. Intern. Med. 137, 381-433). Moreover, HIV is capable of developing high resistance to all known inhibitors that are used in the present HAART drug regimens, and the transmission of drug-resistant strains is being increasingly reported (reviewed in: Wensing, A. M. & Boucher, C. A. (2003) AIDS Rev. 5, 140-155).

Therefore, there is a need for advanced antiretroviral strategies that provide novel therapy options, particularly for patients infected with multiple HAART-resistant HIV strains.

### The present invention

The present invention relates to the use of compounds having the general formula 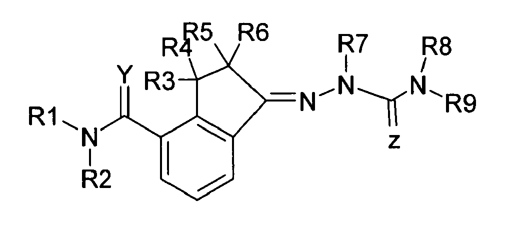 or pharmaceutically acceptable salts thereof for the preparation of a pharmaceutical preparation for treating viral infections, in particular retroviral infections.

In the formula above, R1, R2, R3, R4, R5, R6, R7, R8, R9, Y and Z are defined as follows:
R1, R2, R3, R4, R5, R6, R7, R8, and R9 are each independently selected from H or alkyl,
Y is O, S, NH or NR,
   and
Z is O, S, NH or NR,
wherein R is hydrogen or alkyl.

Alkyl refers to a CₙHₙ₊₂ radical, preferably having 1 to 7 carbon atoms. Alkyl is, for example, n-propyl, isopropyl, n-butyl, isobutyl, sec-butyl, tert.-butyl, n-pentyl, neopentyl, n-hexyl or n-heptyl, preferably ethyl and most especially methyl.

A particularly preferred compound of the invention is 2-[4-(aminoiminomethyl)-2,3-dihydro-1H-inden-1-ylidene]-hydrazinecarboximidamide (C₁₁H₁₄N₆) 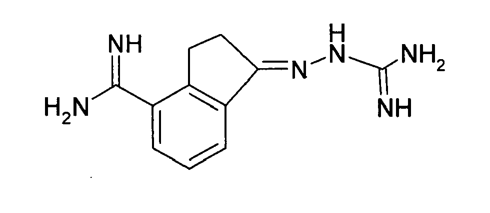 including its pharmaceutically acceptable salts and (chelate) complexes.

Salts of compounds according to the invention are especially pharmaceutically acceptable non-toxic salts. For example, compounds of formula I having basic groups may form acid addition salts, for example with inorganic acids, such as hydrochloric acid, sulfuric acid or phosphoric acid, or with suitable organic carboxylic or sulfonic acids, for example acetic acid, fumaric acid or methanesulfonic acid, or, for example, with amino acids, such as arginine or lysine. When several basic groups are present, mono- or poly-salts may be formed. Compounds of formula I having an acid group, for example carboxy, and a basic group, for example amino, may also be, for example, in the form of internal salts, i.e. in zwitterionic form, or part of the molecule may be in the form of an internal salt and another part may be in the form of a normal salt. Particularly preferred is the dihydrochloride monohydrate of compound I, namely 2-[4-(aminoiminomethyl)-2,3-dihydro-1H-inden-1-ylidene]-hydrazinecarboximidamide dihydrochloride, C₁₁H₁₄N₆ · 2 HCl). In connection with the present invention, the salts and complexes, in particular the afore-mentioned dihydrochloride monohydrate are explicitly included when referring to 2-[4-(aminoiminomethyl)-2,3-dihydro-1H-inden-1-ylidene]-hydrazinecarboximidamide, even if not explicitely mentioned.

According to a preferred embodiment of the invention, the retroviral infections are selected from lentiviral or oncoretroviral infections. Lentiviruses include Human immunodeficiency virus (HIV), Equine infectious anemia virus (EIAV), Maedi-visna virus (MVV), Caprine arthritis encephalitis virus (CAEV), Bovine immunodeficiency virus (BIV), Feline immunodeficiency virus (FIV) and Simian immunodeficiency virus (SIV). In particular, HIV includes HIV-1. Oncoretroviruses include Human T cell leukemia virus (HTLV) and Bovine leukemia virus (BLV).

In the context with the present invention it was surprisingly found that compounds of the above formula exhibit excellent activity against various viruses, in particular against HIV-1, and can therefore be used for treatment of virally induced infections, including opportunistic infections, and also diseases associated with such infections. In particular, the compounds demonstrate pronounced antiviral effects against the above mentioned retroviruses.

According to a specific embodiment of the invention, the compound is 2-[4-(aminoiminomethyl)-2,3-dihydro-1H-inden-1-ylidene]-hydrazinecarboximidamide, and the retroviral infection is an HIV infection, in particular HIV-1.

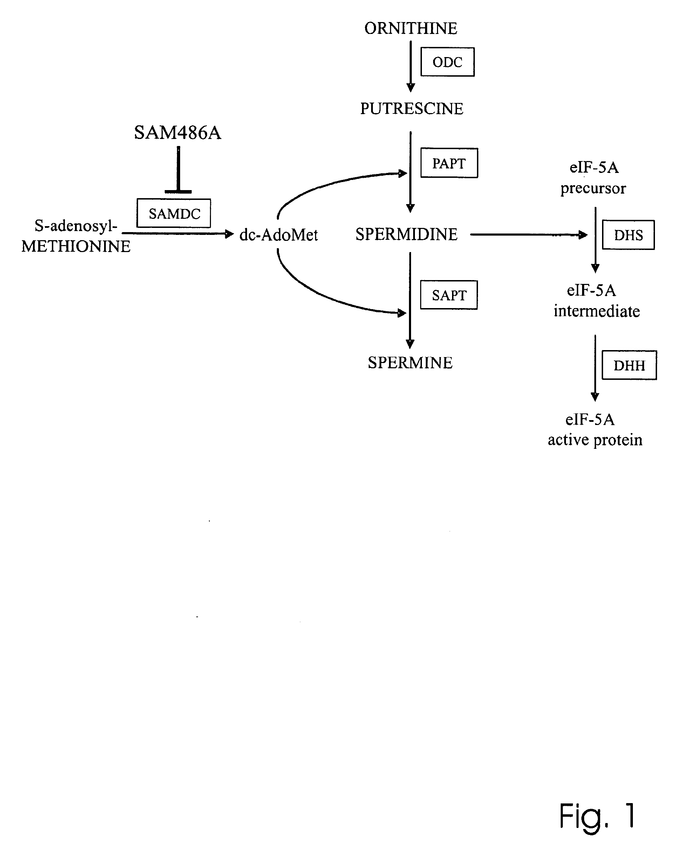

In the context with the present invention, pronounced antiretroviral activity (in particular against HIV) is demonstrated in particular for 2-[4-(aminoiminomethyl)-2,3-dihydro-1H-inden-1-ylidene]-hydrazinecarboximidamide , which has been previously identified to be an efficient inhibitor of cellular S-adenosylmethionine decarboxylase (SAMDC) (Regenass, U., Mett, H., Stanek, J., Mueller, M., Kramer, D. & Porter, C. W. (1994) Cancer Res. 54, 3210-3217; Kramer, D., Mett, H., Evans, A., Regenass, U., Diegelman, P. & Porter, C. W. (1995) J. Biol. Chem. 270, 2124-2132; Tolbert, W. D., Ekstrom, J. L., Mathews, I. I., Secrist, J. A., III, Kapoor, P., Pegg, A. E. & Ealick, S. E. (2001) Biochemistry 40, 9484-9494). SAMDC provides decarboxylated-S-adenosylmethionine (dc-AdoMet; see Fig. 1) for synthesis of spermidine and spermine (Pegg, A. E. & McCann, P. P. (1992) Pharmacol. Ther. 56, 359-377; Tolbert, W. D., Ekstrom, J. L., Mathews, I. I., Secrist, J. A., III, Kapoor, P., Pegg, A. E. & Ealick, S. E. (2001) Biochemistry 40, 9484-9494; Shantz, L. M. & Pegg, A. E. (1998) Methods Mol. Biol. 79, 45-49) and is therefore a central enzyme of the polyamine biosynthesis pathway (Pegg, A. E., Xiong, H., Feith, D. J. & Shantz, L. M. (1998) Biochem. Soc. Trans. 26, 580-586). 2-[4-(aminoiminomethyl)-2,3-dihydro-1H-inden-1-ylidene]-hydrazinecarboximidamide is currently being tested in phase II clincial studies for treatment of metastatic melanoma and refractory or relapsed non-Hodgkin's lymphoma (Millward, M. J., Joshua, A., Kefford, R., Aamdal, S., Thomson, D., Hersey, P., Toner, G. & Lynch, K. (2005) Invest New Drugs 23, 253-256; Pless, M., Belhadj, K., Menssen, H. D., Kern, W., Coiffier, B., Wolf, J., Herrmann, R., Thiel, E., Bootle, D., Sklenar, I. et al. (2004) Clin. Cancer Res. 10, 1299-1305).

In view of the results obtained in accordance with the invention, in particular in view of the low or lacking toxicity of the compounds mentioned herein, treatment regimens are provided which make possible therapeutic strategies, in particular in situations where otherwise multiple antiretroviral drug (HAART)-resistant viruses are involved.

The present invention is described below by way of examples, figures and tables, which are intended to illustrate but not to limit the invention. Specifically, the antiviral activity is shown for HIV-1 by investigating the biosynthesis pathway of eukaryotic initiation factor 5A (eIF-5A), which is a critical cellular cofactor of the retroviral Rev regulatory protein (Hauber, J. (2001) Curr. Top. Microbiol. Immunol. 259:55-76), by using one of the most preferred compounds of the invention, 2-[4-(aminoiminomethyl)-2,3-dihydro-1H-inden-1-ylidene]-hydrazinecarboximidamide dihydrochloride (designated as "SAM486A"). The here exemplarily shown HIV-1 inhibitory effect of 2-[4-(aminoiminomethyl)-2,3-dihydro-1H-inden-1-ylidene]-hydrazinecarboximidamide dihydrochloride is also observed with other viruses, in particular retroviruses (encoding a Rev-like activity). Compounds falling under the general formula other than 2-[4-(aminoiminomethyl)-2,3-dihydro-1H-inden-1-ylidene]-hydrazinecarboximidamide can be used as well.

### Figure Legends

### Fig. 1.

Schematic representation of the polyamine and eIF-5A biosynthesis pathway. Putrescine is formed by decarboxylation of ornithine, a reaction that is catalyzed by ornithine decarboxylase (ODC). Spermidine is synthesized by the action of putrescine aminopropyl transferase (PAPT). The aminopropyl group is derived from decarboxylated-S-adenosylmethionine (dc-AdoMet), which is provided by S-adenosylmethionine decarboxylase (SAMDC). 2-[4-(aminoiminomethyl)-2,3-dihydro-1H-inden-1-ylidene]-hydrazinecarboximidamide dihydrochloride is a potent inhibitor of SAMDC (Regenass, U., Mett, H., Stanek, J., Mueller, M., Kramer, D. & Porter, C. W. (1994) Cancer Res. 54, 3210-3217). Spermine is formed by addition of an aminopropyl group to spermidine by enzymatic action of spermidine aminopropyl transferase (SAPT). The biological activity of the eukaryotic initiation factor 5A (eIF-5A) depends on its hypusine modification, a spermidine-dependent posttranslational reaction that is catalyzed by subsequent action of the two enzymes deoxyhypusine synthase (DHS) and deoxyhypusine hydroxylase (DHH).

### Fig. 2.

Analysis of cellular toxicity. (A) Cell viability assay. PM1 cells were cultured for 17 days in various concentrations of 2-[4-(aminoiminomethyl)-2,3-dihydro-1H-inden-1-ylidene]-hydrazinecarboximidamide dihydrochloride or medium alone (Control). Cellular metabolic activity was tested by alamarBlue^{™} assay (Serotec) at the indicated days. *(B)* Cell-cycle analysis. PM1 cells were cultured for 7 days in presence of 0.4 µM 2-[4-(aminoiminomethyl)-2,3-dihydro-1H-inden-1-ylidene]-hydrazinecarboximidamide dihydrochloride or medium alone (Control). FACS-analysis was performed by DNA-staining with propidiumiodide. (*C*) Apoptosis-assay. PBMCs from a healthy donor were cultured for 12 days in the presence of 0.4 µM 2-[4-(aminoiminomethyl)-2,3-dihydro-1H-inden-1-ylidene]-hydrazinecarboximidamide dihydrochloride or medium alone (Control). Subsequently, apoptotic cells were assayed by FACS using FITC-coupled annexin V.

### Fig. 3.

Inhibition of HIV-1 BaL replication by 2-[4-(aminoiminomethyl)-2,3-dihydro-1H-inden-1-ylidene]-hydrazinecarboximidamide dihydrochloride (*A*) HIV-1 BaL-infected PM1 cells were cultured in the presence of the indicated concentrations of 2-[4-(aminoiminomethyl)-2,3-dihydro-1H-inden-1-ylidene]-hydrazinecarboximidamide dihydrochloride or medium alone (Control). The culture medium was changed at day 3 and 6 post-infection and the cells were split 1:1. p24^{Gag} antigen levels and cell viabilities were determined at day 6 and 9 post-infection. The percentage of inhibition of virus replication in the drug-treated cell culture, as compared to the untreated controls is shown in the upper panel. Cell viabilities from the same cultures were determined by alamarBlue^{™} assay (Serotec) and is shown in the lower panel. (*B*) Gag-specific Western-blot analysis. Total cellular protein extracts were prepared at day 6 post-infection from a culture treated with 2-[4-(aminoiminomethyl)-2,3-dihydro-1H-inden-1-ylidene]-hydrazinecarboximidamide dihydrochloride (0.4 µM) and from an untreated (Control) culture and subjected to immune blot analysis using HIV-1 p24-specific or α-tubulin-specific (protein loading control) antibodies. (*C*) Analysis of de novo infection. PM1 cells were cultured for 10 days in the presence or absence of 0.4 µM 2-[4-(aminoiminomethyl)-2,3-dihydro-1H-inden-1-ylidene]-hydrazinecarboximidamide dihydrochloride and subsequently infected with HIV-1 BaL. 3.0 µg of total genomic DNA, which was isolated from an uninfected culture (lane 1) or from the infected cultures at 90, 120 and 150 minutes post-infection (lane 2 to lane 4, respectively), was directly amplified using HIV-1 specific primer pairs recognizing extrachromosomal 1-LTR and 2-LTR circular pre-integration DNA (PID) as described in Hauber, I., Harrer, T., Löw, P., Schmitt, M., Schwingel, E. & Hauber, J. (2000) AIDS 14, 2619-2621. *(D)* Detection of integrated proviral DNA. Total genomic DNA was isolated from uninfected (lane 1) PM1 cells and from cells 3 days after infection (lane 2). 2.5 µg of the respective DNA was PCR amplified using primer pairs specific for the HIV-1 BaL LTR and chromosomal *Alu* repeats.

### Fig. 4.

Inhibition of a multiple HAART-resistant HIV-1 isolate. (A) PM1 cells were infected with a antiretroviral drug-resistant virus and cultured in the presence of 0.4 µM 2-[4-(aminoiminomethyl)-2,3-dihydro-1H-inden-1-ylidene]-hydrazinecarboximidamide dihydrochloride or medium alone (Control). Culture medium was changed every third day and cells were split 1:1. Cell viabilities and p24 antigen levels were determined at the indicated days. The percentage of inhibition of virus replication in the drug-treated culture as compared with replication in the untreated control culture is shown in the upper panel. The respective virus growth curves as measured by p24 antigen levels in the culture supernatants are shown in the lower panel. n/a; not applicable. (*B*) Cellular toxicities were determined in the same cultures by alamarBlue^{™} assay (Serotec) for metabolic activity.

### Fig. 5.

2-[4-(aminoiminomethyl)-2,3-dihydro-1H-inden-1-ylidene]-hydrazinecarboximidamide dihydrochloride prevents hypusine-formation in eIF-5A. PM1 and HeLaCD4-CAT cells were metabolically labeled with [¹⁴C]putrescine in the presence or absence (Control) of 0.4 µM 2-[4-(aminoiminomethyl)-2,3-dihydro-1H-inden-1-ylidene]-hydrazinecarboximidamide dihydrochloride(*A*) Two-dimensional gel electrophoresis of total protein extracts prepared from PM1 cells. Protein separation was performed using equal amounts of radiolabeled extracts, by isoelectric focusing in the first dimension on an immobilized linear pH gradient (IPG) from pH 4.0 to 7.0, which was followed by SDS-PAGE separation in the second dimension. Only molecular mass of < 25.0 kDa is shown in the autoradiographies. Molecular mass standards are indicated in kDa on the right. (*B*) Equal amounts of cell extracts treated with 2-[4-(aminoiminomethyl)-2,3-dihydro-1H-inden-1-ylidene]-hydrazinecarboximidamide dihydrochloride (lane 1) and untreated (Control; lane 2) HeLaCD4-CAT cell extracts were subjected to eIF-5A-specific immunoprecipitation and analyzed by 14 % SDS-PAGE and autoradiography (upper panel). Coomassie blue-staining of the gel served as protein loading control (lower panel).

### Fig. 6.

Effect of 2-[4-(aminoiminomethyl)-2,3-dihydro-1H-inden-1-ylidene]-hydrazinecarboximidamide dihydrochloride on HIV-1 trans-activation. (*A*) Analysis of Rev *trans*-activation using the Rev-responsive pCMVgagLucRRE reporter plasmid. HeLaCD4-CAT cells were cultured for 5 days in 0.4 µM 2-[4-(aminoiminomethyl)-2,3-dihydro-1H-inden-1-ylidene]-hydrazinecarboximidamide dihydrochloride or medium alone (Control) and subsequently cotransfected with pCMVgagLucRRE, a Rev-expressing vector (+Rev) or the respective parental plasmid (Rev-deficient control; -Rev) and the constitutive internal control vector pBC12/CMV/pGal. Rev activity was determined at 48 hours post-transfection by luciferase reporter assay. All luciferase values were adjusted for transfection efficiency by determining the level of β-galactosidase in each culture. CMV-IE, cytomegalovirus immediate early promoter; pA, polyadenylation site; SD, splice donor; SA, splice acceptor; RRE, Rev reponse element. (B) Analysis of Tat *trans*-activation using the Tat-responsive pBC12/HIV/CAT reporter construct. HeLaCD4-CAT cells were cultured in 2-[4-(aminoiminomethyl)-2,3-dihydro-1H-inden-1-ylidene]-hydrazinecarboximidamide dihydrochloride as before and subsequently cotransfected with pBC12/HIV/CAT, a Tat-expressing vector (+Tat) or the parental plasmid (Tat-deficient control; -Tat) and the internal control vector pBC12/CMV/βGal. Tat activity was determined at 48 hours post-transfection by CAT detection. All CAT values were adjusted for transfection efficiency by determining the level of β-galactosidase in each culture. HIV-LTR, HIV long terminal repeat promoter.

### Fig. 7.

Specific inhibition of the HIV-1 Rev pathway by 2-[4-(aminoiminomethyl)-2,3-dihydro-1H-inden-1-yliderie]-hydrazinecarboximidamide dihydrochloride (*A*) Schematic representation of HIV-derived subgenomic *gag-pol* constructs (Swanson, C. M., Puffer, B. A., Ahmad, K. M., Doms, R. W. & Malim, M. H. (2004) EMBO J. 23, 2632-2640). Transcripts expressed from GPV-RRE access the CRM1/exporti nuclear export pathway. Messages expressed from GPV-4xCTE are transported across the nuclear envelope via the NXF1/TAP pathway. CMV-IE, cytomegalovirus immediate early promoter; pA, polyadenylation site; SD, splice donor; SA, splice acceptor; RRE, Rev reponse element; CTE, constitutive transport element. (*B*) HeLaCD4-CAT cells were cultured for 5 days in 0.4 µM 2-[4-(aminoiminomethyl)-2,3-dihydro-1H-inden-1-ylidene]-hydrazinecarboximidamide dihydrochloride or medium alone (Control) as before, followed by cotransfection with GVP-RRE and a Rev-expressing vector (+Rev) or a respective parental plasmid as a Rev-deficient control (-Rev) and a constitutive internal control vector pBC12/CMV/SEAP. Likewise, cultures were transfected with GVP-4xCTE, a negative control plasmid (to maintain input DNA levels) and the constitutive internal control vector pBC12/CMV/SEAP. At 48 hours post-transfection cell supernatants were assayed for secreted alkaline phosphatase (SEAP) and p24 antigen levels. In addition, p55^{Gag}- and α-tubulin-specific (protein loading control) Western-blot analyses were performed from representative cell cultures at day 6 after infection.

### EXAMPLES

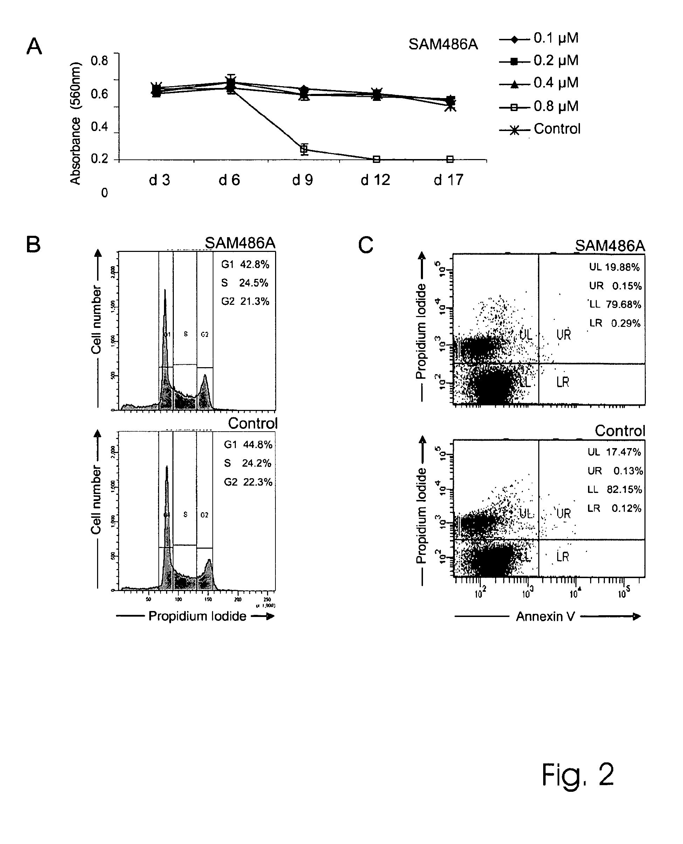

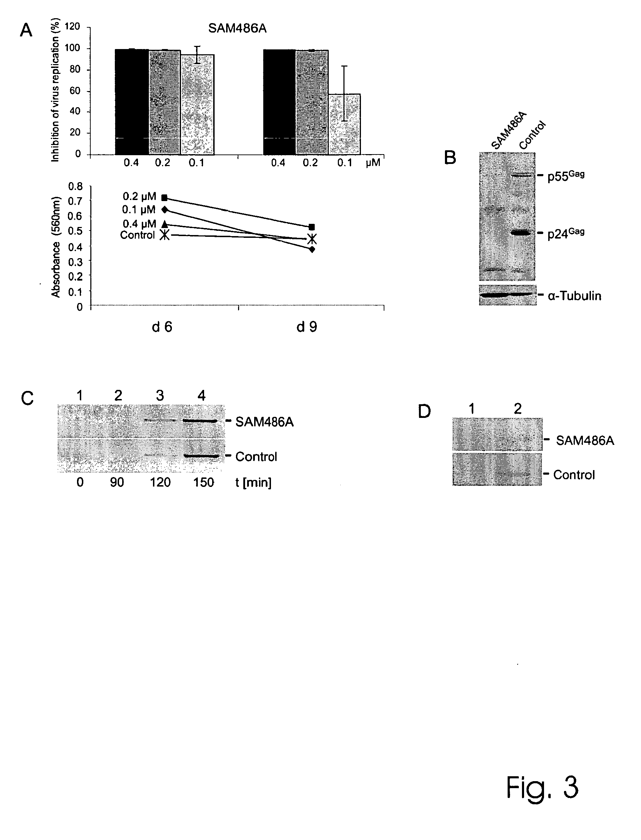

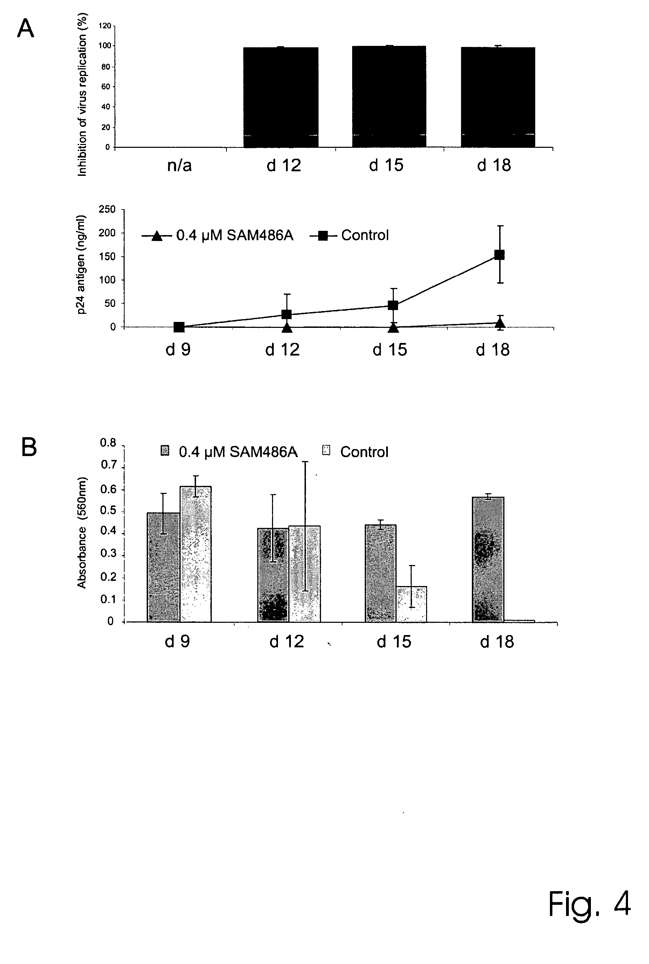

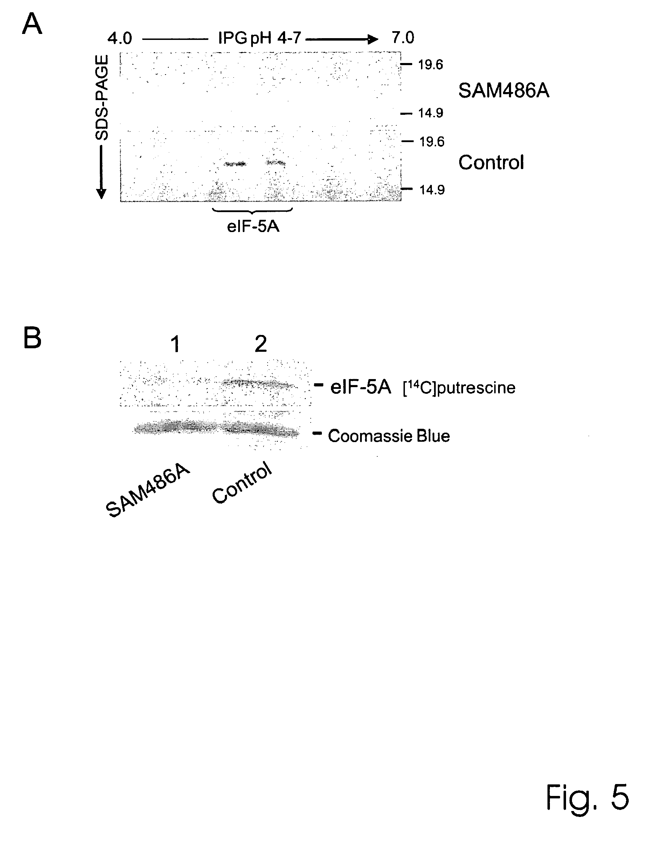

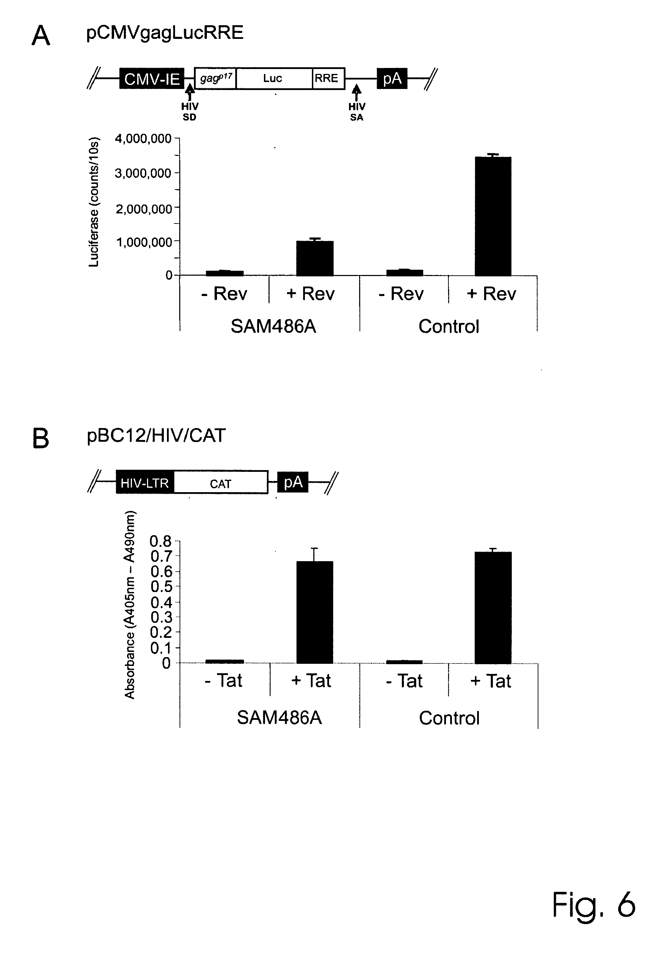

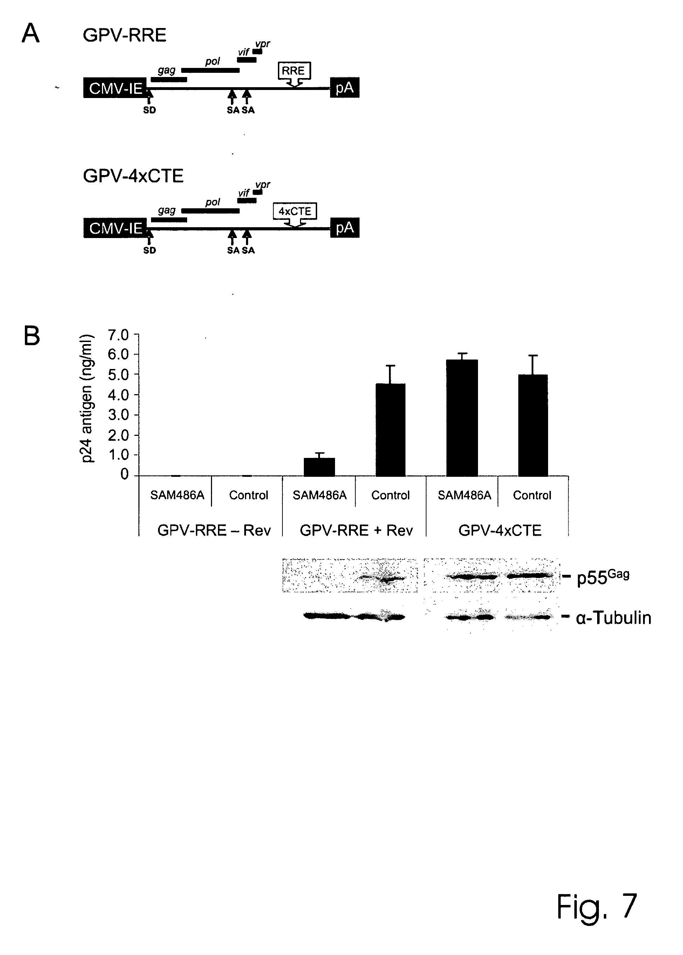

In the context of the present invention, it was possible to demonstrate pronounced antiviral effects in cultures treated with 2-[4-(aminoiminomethyl)-2,3-dihydro-1H-inden-1-ylidene]-hydrazinecarboximidamide dihydrochloride , which were infected with the slow-replicating, macrophage-tropic laboratory strain HIV-1 BaL (Fig. 3). Likewise, the formation of progeny viruses was also inhibited by 2-[4-(aminoiminomethyl)-2,3-dihydro-1H-inden-1-ylidene]-hydrazinecarboximidamide dihydrochloride when the rapidly replicating T cell-tropic virus isolate FE9 was analyzed (Fig. 4), which is characterized by high-level resistance to the full array of reverse transcriptase and protease inhibitors (Hauber, I., Bevec, D., Heukeshoven, J., Krätzer, F., Horn, F., Choidas, A., Harrer, T. & Hauber, J. (2005) J. Clin. Invest 115, 76-85). It is important to note, that this virus inhibition was not due to general drug-induced cellular toxicity, since the applied concentration of 2-[4-(aminoiminomethyl)-2,3-dihydro-1H-inden-1-ylidene]-hydrazinecarboximidamide dihydrochloride (0.4 µM) perfectly blocked virus replication but did not induce deleterious effects on cell cycle progression, cell death and cellular metabolism (Fig. 2). Interestingly, in the phase II clinical studies mentioned above, patients were treated with 100 mg/m² 2-[4-(aminoiminomethyl)-2,3-dihydro-1H-inden-1-ylidene]-hydrazinecarboximidamide dihydrochloride (Millward, M. J., Joshua, A., Kefford, R., Aamdal, S., Thomson, D., Hersey, P., Toner, G. & Lynch, K. (2005) Invest New Drugs 23, 253-256; Pless, M., Belhadj, K., Menssen, H. D., Kern, W., Coiffier, B., Wolf, J., Herrmann, R., Thiel, E., Bootle, D., Sklenar, I. et al. (2004) Clin. Cancer Res. 10, 1299-1305), which corresponds to an approximate drug concentration of >100 µM, well boave the concentration that demonstrated pronounced anti-HIV activity in the present study. This shows that inclusion of 2-[4-(aminoiminomethyl)-2,3-dihydro-1H-inden-1-ylidene]-hydrazinecarboximidamide treatment regimens into future anti-HIV therapies may be indeed tolerable. Clearly, additional preclinical and clinical analyses are required before such an approach may become a therapeutic reality. Nevertheless, the present findings further support the notion that the targeting of host-specific components may provide a strategy to block replication of otherwise multiple antiretroviral drug (HAART)-resistant viruses.

### Materials and Methods

**Compound.** For interference with the polyamine biosynthesis pathway by cellular depletion of spermidine and spermine, the low molecular weight experimental drug 2-[4-(aminoiminomethyl)-2,3-dihydro-1H-inden-1-ylidene]-hydrazinecarboximidamide dihydrochloride (Regenass, U., Mett, H., Stanek, J., Mueller, M., Kramer, D. & Porter, C. W. (1994) Cancer Res. 54, 3210-3217) was used. 2-[4-(aminoiminomethyl)-2,3-dihydro-1H-inden-1-ylidene]-hydrazinecarboximidamide dihydrochloride is a potent inhibitor of S-adenosylmethionine decarboxylase (SAMDC) and can be prepared according to the method of Stanek et al. (J. Med. Chem. (1993) 36:2168-2171).

A stock solution of 10 mM 2-[4-(aminoiminomethyl)-2,3-dihydro-1H-inden-1-ylidene]-hydrazinecarboximidamide dihydrochloride was prepared in sterile water (stored at -20°C) and diluted further to working concentration using cell culture medium.

**Analysis of Cellular Toxicity**. Cell viability was analyzed by measuring cellular metabolic activity using the alamarBlue^{™} redox indicator according to the manufacturer's protocol (Serotec). For cell-cycle analysis PM1 cells were cultured for 7 days in presence of 0.4 µM 2-[4-(aminoiminomethyl)-2,3-dihydro-1H-inden-1-ylidene]-hydrazinecarboximidamide dihydrochloride or medium alone (Control). FACS-analysis was performed by DNA-staining with propidiumiodide (CycleTest™ Plus; Becton Dickinson). For analysis of apoptosis primary bone marrow cells (PBMCs) from a healthy donor were cultured in 0.4 µM 2-[4-(aminoiminomethyl)-2,3-dihydro-1H-inden-1-ylidene]-hydrazinecarboximidamide dihydrochloride or medium alone (Control) for 12 days. Subsequently, apoptotic cells were assayed by FACS-analysis using FITC-coupled annexin V (Bender MedSystems).

**HIV-1 Infection Experiments.** HIV-1 infection using the macrophage-tropic strain HIV-BaL (Gartner, S., Markovits, P., Markovitz, D. M., Kaplan, M. H., Gallo, R. C. & Popovic, M. (1986) Science 233, 215-219) or the multidrug-resistant T-cell tropic HIV-1 isolate FE9 (Hauber, I., Bevec, D., Heukeshoven, J., Krätzer, F., Horn, F., Choidas, A., Harrer, T. & Hauber, J. (2005) J. Clin. Invest 115, 76-85) were routinely performed in PM1 cells (HIV-1 BaL and PM1 cells were obtained from the NIH AIDS Research and Reference Reagent Program). The drug-resistance confering mutations in the FE9 isolate and the respective phenotypic drug sensitivity profile were described previously (Hauber, I., Bevec, D., Heukeshoven, J., Krätzer, F., Horn, F., Choidas, A., Harrer, T. & Hauber, J. (2005) J. Clin. Invest 115, 76-85).

PM1 cells were cultured in RPMI 1640 medium containing 10% fetal calf serum (Pansystems GmbH) and antibiotics (penicillin and streptomycin) and infected with the virus strains exactly as described elsewhere (Hauber, I., Bevec, D., Heukeshoven, J., Krätzer, F., Horn, F., Choidas, A., Harrer, T. & Hauber, J. (2005) J. Clin. Invest 115, 76-85). After infection, cells were washed twice with PBS without Ca²⁺ and Mg²⁺ to avoid false-positive p24 antigen determination. Cells were resuspended and identical aliquots of 5 x 10⁵ per ml of infected cells were further cultured in RPMI medium in the presence of various concentrations of 2-[4-(aminoiminomethyl)-2,3-dihydro-1H-inden-1-ylidene]-hydrazinecarboximidamide dihydrochloride or medium (solution control) for the calculation of the inhibition of virus replication. Culture medium was changed and cells were split 1:1 at the indicated days post-infection. At the same time cell viability (measured by alamarBlue^{™} assay) and p24 antigen levels in the supernatant (Innotest HIV p24 Antigen mAb; Innogenetics N. V.) were determined. In addition, total cellular protein extracts were subjected to Western blot analyses using the HIV-1 p24 mAb 183-H12-5C (NIH AIDS Research and Reference Reagent Program) (Chesebro, B., Wehrly, K., Nishio, J. & Perryman, S. (1992) J. Virol. 66, 6547-6554) or an α-tubulin-specific (protein loading control) monoclonal antibody (Sigma-Aldrich).

**Analysis of De Novo Infection and Proviral Integration**. De novo infection was examined by PCR of extrachromosomal circular viral DNA. For this, PM1 cells were cultured for 10 days in presence of 0.4 µM 2-[4-(aminoiminomethyl)-2,3-dihydro-1H-inden-1-ylidene]-hydrazinecarboximidamide dihydrochloride or medium alone (Control) and subsequently infected with HIV-1 BaL. Total genomic DNA was isolated from uninfected cells and from the infected cells at 90, 120 and 150 min post-infection. 3.0 µg of these genomic DNAs were directly amplified using HIV-1 specific primer pairs recognizing extrachromsomal 1- long terminal repeat (LTR) and 2-LTR circular pre-integration DNA (PID) as previously described (Hauber, I., Harrer, T., Löw, P., Schmitt, M., Schwingel, E. & Hauber, J. (2000) AIDS 14, 2619-2621). Detection of integrated proviral DNA was assayed at day 3 post-infection by a nested PCR using 2.5 µg of genomic DNA and primer pairs complementary to the HIV-1 BaL long-terminal repeat (LTR) and chromosomal Alu repeats: LTR outer/forward, 5'-GAGCAGTATCTCGAGACCTGG-3' (SEQ ID NO:1); human *Alu* outer/reverse, 5'-TGCTGGGATTACAGGCGTGAG-3' (SEQ ID NO: 2). LTR nested/forward, 5'- GAGCAATCACAAGTAGCAATACAGC-3' (SEQ ID NO:3) ; LTR nested/reverse, 5'- CCTTGTAGAAAGCTCGGTGTCAG-3' (SEQ ID NO:4). The amplification profile involved 30 cycles of denaturation at 95°C for 1 minute, primer annealing at 54°C for 1 minute and primer extension at 72°C for 3 minutes for the first round of PCR, followed by 40 cycles for the nested PCR.

**Two-Dimensional Gel Electrophoresis and Immunoprecipitation Analysis.** To analyze the effects of 4-(aminoiminomethyl)-2,3-dihydro-1H-inden-one-diaminomethylene-hydrazone on hypusine-modification of eIF-5A by two-dimensional gel electrophoresis, 6 x 10⁶ PM1 cells were were metabolically labeled for 12 hours using 25 µCi [1,4-¹⁴C]putrescine dihydrochloride (metabolic precursor of spermidine; 107 mCi/mmol; Amersham) in presence or absence of 0.4 µM 2-[4-(aminoiminomethyl)-2,3-dihydro-1H-inden-1-ylidene]-hydrazinecarboximidamide dihydrochloride. Gel analysis was performed by autoradiography as previously described (Hauber, I., Bevec, D., Heukeshoven, J., Krätzer, F., Horn, F., Choidas, A., Harrer, T. & Hauber, J. (2005) J. Clin. Invest 115, 76-85). For immunoprecipitation of eIF-5A, 2 x 10⁶ HeLaCD4-CAT cells (obtained through the NIH AIDS Research and Reference Reagent Program from B.K.Felber and G.N.Pavlakis) were metabolically labeled as above. The various cellular extracts were prepared and analyzed using a rabbit polyclonal anti-eIF-5A antibody (Schatz, O., Oft, M., Dascher, C., Schebesta, M., Rosorius, O., Jaksche, H., Dobrovnik, M., Bevec, D. & Hauber, J. (1998) Proc. Natl. Acad. Sci. U. S. A 95, 1607-1612) as previously described in detail (Hauber, I., Bevec, D., Heukeshoven, J., Krätzer, F., Horn, F., Choidas, A., Harrer, T. & Hauber, J. (2005) J. Clin. Invest 115, 76-85).

**Transfection Experiments and Plasmids**. HeLaCD4-CAT cells were transiently transfected using Lipofectamine and Plus reagent according to the manufacturer's protocol (Invitrogen Corp.). Rev trans-activation was investigated by cotransfection of 2.5 x 10⁵ HeLaCD4-CAT cells with 0.6 µg of pCMVgagLucRRE (31) and 0.3 µg of pBC12/CMV/βGal DNA (Ruhl, M., Himmelspach, M., Bahr, G. M., Hammerschmid, F., Jaksche, H., Wolff, B., Aschauer, H., Farrington, G. K., Probst, H., Bevec, D. et al. (1993) J. Cell Biol. 123, 1309-1320), together with 0.3 µg of either pcRev (Malim, M. H., Hauber, J., Fenrick, R. & Cullen, B. R. (1988) Nature 335, 181-183) or the parental pBC12/CMV (Cullen, B. R. (1986) Cell 46, 973-982) plasmid. At 48 hours post-transfection, cellular lysates were prepared and the levels of β-galactosidase (transfection efficiency control) and luciferase were assayed by ELISA (Roche Applied Science) or luminescence (Promega), respectively. Likewise, Tat *trans-*activation was investigated by cotransfection of 2.5 x 10⁵ HeLaCD4-CAT cells with 1.0 µg of pBC12/HIV/CAT DNA (Berger, J., Hauber, J., Hauber, R., Geiger, R. & Cullen, B. R. (1988) Gene 66, 1-10) and 0.6 µg of pBC12/CMV/βGal DNA, together with 0.5 µg of either pcTat (Malim, M. H., Hauber, J., Fenrick, R. & Cullen, B. R. (1988) Nature 335, 181-183) or the parental pBC12/CMV plasmid. At 48 hours post-transfection, cellular lysates were prepared and the levels of β-galactosidase (transfection efficiency control) and chloramphenicol acetyltransferase (CAT) were assayed by ELISA (Roche Applied Science). To analyze HIV-1 *gag-pol* mRNA export in HeLaCD4-CAT cells treated with 2- [4- (aminoiminomethyl) -2,3-dihydro-1H-inden-1-ylidenel-hydrazinecarboximidamide dihydrochloride and untreated HeLaCD4-CAT cells, the subgenomic vectors GPV-RRE and GPV-4xCTE were used (kindly provided by M. Malim, Department of Infectious Diseases, King's College London, UK) (Swanson, C. M., Puffer, B. A., Ahmad, K. M., Doms, R. W. & Malim, M. H. (2004) EMBO J. 23, 2632-2640). For this, 2.5 x 10⁵ cells were cotransfected with either 0.5 µg GPV-RRE or 0.5 µg GPV-4xCTE together with 0.25 µg pBC12/CMV/SEAP (transfection efficiency control) (Berger, J., Hauber, J., Hauber, R., Geiger, R. & Cullen, B. R. (1988) Gene 66, 1-10) and 0.25 µg of either pcRev or the parental pBC12/CMV plasmid. At 48 hours post-transfection cell supernatants were assayed for secreted alkaline phosphatase (SEAP) (Berger, J., Hauber, J., Hauber, R., Geiger, R. & Cullen, B. R. (1988) Gene 66, 1-10) and p24 antigen.

## Claims

1. Use of compounds having the general formula 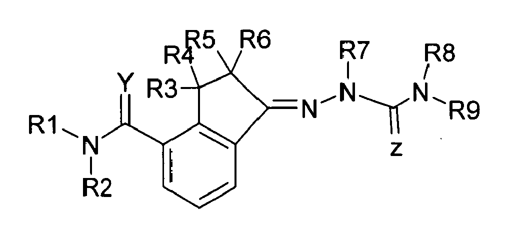 or pharmaceutically acceptable salts thereof for the preparation of a pharmaceutical preparation for treating viral infections,
wherein R1, R2, R3, R4, R5, R6, R7, R8, and R9 are each independently selected from H or alkyl,
Y is O, S, NH or NR, and
Z is O, S, NH or NR,
wherein R is hydrogen or alkyl.

2. Use according to claim 1, wherein the viral infections are retroviral infections.

3. Use according to claim 2, wherein the retroviral infections are lentiviral or oncoretroviral infections.

4. Use according to claim 3, wherein the lentiviral infection is an infection with Human immunodeficiency virus (HIV), Equine infectious anemia virus (EIAV), Maedi-visna virus (MVV), Caprine arthritis encephalitis virus (CAEV), Bovine immunodeficiency virus (BIV), Feline immunodeficiency virus (FIV) and Simian immunodeficiency virus (SIV).

5. Use according to claim 3, wherein the oncoretroviral infection is an infection with Human T cell leukemia virus (HTLV) and Bovine leukemia virus (BLV).

6. Use according to claims 1 to 5, wherein the compound is 2-[4-(aminoiminomethyl)-2,3-dihydro-1H-inden-1-ylidene]-hydrazinecarboximidamide dihydrochloride and wherein the HIV is HIV-1.


SEQUENCE LISTING

<110>  Heinrich-Pette-Institut

<120>  Use of specific hydrazone compounds for the treatment of
       viral infections

<130>  P 70829

<160>  4

<170>  PatentIn version 3.3

<210>  1
<211>  21
<212>  DNA
<213>  Artificial

<220>
<223>  Primer

<400>  1
gagcagtatc tcgagacctg g                                                21

<210>  2
<211>  21
<212>  DNA
<213>  Artificial

<220>
<223>  Primer

<400>  2
tgctgggatt acaggcgtga g                                                21

<210>  3
<211>  25
<212>  DNA
<213>  Artificial

<220>
<223>  Primer

<400>  3
gagcaatcac aagtagcaat acagc                                            25

<210>  4
<211>  23
<212>  DNA
<213>  Artificial

<220>
<223>  Primer

<400>  4
ccttgtagaa agctcggtgt cag                                              23